# EUROPÄISCHE PATENTSCHRIFT

(11) **EP 0 786 263 B1**
(45) Veröffentlichungstag und Bekanntmachung des Hinweises auf die Patenterteilung: **11.09.2002**
(21) Anmeldenummer: 97100520.2
(22) Anmeldetag: 15.01.1997
(51) Int. Cl.: A61M 11/06, B05B 7/00

(54) **Vernebler**
Nebulizer
Nébuliseur

(30) Priorität: 25.01.1996 DE 19602628
(43) Veröffentlichungstag der Anmeldung: 30.07.1997
(73) Patentinhaber: PARI GmbH Spezialisten für effektive Inhalation, 82319 Starnberg (DE)
(72) Erfinder: Wunderlich, Erik, 82205 Gilching (DE); Knoch, Martin, Dr., 82335 Berg (DE); Waldner, Robert, 86971 Peiting (DE)
(74) Vertreter: Zangs, Rainer E., Dipl.-Ing.

(56) Entgegenhaltungen:
- EP-A- 0 540 775
- EP-A- 0 627 266
- EP-A- 0 653 218

## Beschreibung

Die Erfindung betrifft einen Vernebler, insbesondere zur Anwendung in der Inhalationstherapie, mit den Merkmalen des Oberbegriffs des Patentanspruchs 1.

Ein derartiger Vernebler ist bekannt aus EP-A-0 540 775. Der bei diesem Vernebler erstmals beschriebene Verneblungsraumteiler besitzt zwei Abschnitte, von denen der Prallabschnitt verhindert, daß Flüssigkeitströpfchen direkt von der Verneblerdüse zur Entnahmeöffnung des Vernebelungsraumes gelangen. An dem Prallabschnitt schlagen sich Flüssigkeitströpfchen nieder und werden in den Flüssigkeitsvorrat zurückgeführt. Der Leitabschnitt des Verneblungsraumteilers bewirkt eine Verlängerung des Strömungsweges des Flüssigkeitsnebels bei der Entnahme und sorgt so für eine Homogenisierung und Abtrocknung des Flüssigkeitsnebels.

Mit dem bekannten Vernebler können Aerosole von sehr hoher Qualität erzeugt werden. Wegen des Verneblungsraumteilers existieren bei dem bekannten Vernebler aber Bereiche, in denen sich Flüssigkeitströpfchen niederschlagen, die nicht in den Flüssigkeitsvorrat zurückgeführt werden. Die Menge der Flüssigkeit, die in Form eines Flüssigkeitsnebels oder Aerosols entnommen werden kann, ist daher im Verhältnis zu der Menge, die dazu in den Vernebler eingebracht werden muß, nur unvollständig ausgeschöpft. Mit anderen Worten, die Ausbeute an entnehmbarem Medikamentenaerosol ist klein.

Ausgehend von diesem Stand der Technik liegt der Erfindung die Aufgabe zugrunde, den bekannten Vernebler so weiterzubilden, daß die Ausbeute gesteigert ist.

Gelöst wird diese Aufgabe durch einen Vernebler mit den Merkmalen des Patentanspruchs 1. Vorteilhafte Ausgestaltungen ergeben sich aus den Unteransprüchen.

Im folgenden wird die Erfindung anhand von Ausführungsbeispielen unter Bezugnahme auf die Zeichnungen genauer beschrieben, in denen zeigt:
- Fig. 1: ein Ausführungsbeispiel eines erfindungsgemäßen Verneblers mit integrierter Zerstäuberdüse;
- Fig. 2A bis 2C: einen Bestandteil des Verneblers aus Fig. 1 in unterschiedlichen Ansichten; und
- Fig. 3A bis 3E: verschiedene Ausgestaltungen der Prallflächen.

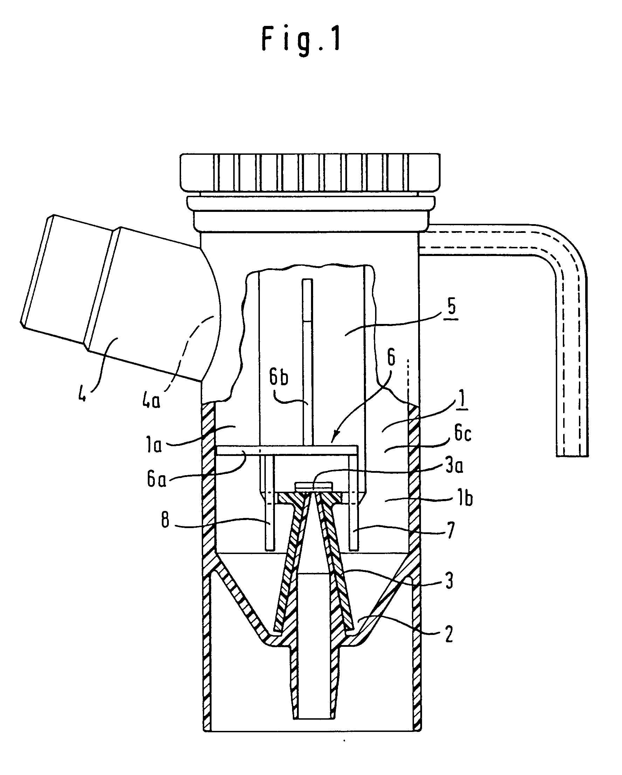

Der in Fig. 1 gezeigte Vernebler besitzt einen zylindrischen Verneblungsraum 1, der nach unten durch einen Flüssigkeitssammelbereich 2 abgeschlossen ist, in den der Flüssigkeitsvorrat eingefüllt wird. Mit Hilfe einer Zerstäuberdüse 3, die mittig in dem zylindrischen Verneblungsraum 1 angeordnet ist, wird der Flüssigkeitsvorrat aus dem Flüssigkeitssammelbereich 2 zerstäubt und auf diese Weise im Bereich der Düsenöffnungen 3a ein Flüssigkeitsnebel erzeugt, der sich im Verneblungsraum 1 ausbreitet. Der Flüssigkeitsnebel wird über einen Ansaugstutzen 4 von einem Patienten dem Vernebler entnommen. Der Ansaugstutzen 4 mündet in eine Öffnung 4a des Verneblungsraumes 1. Um die für die Entnahme des Aerosols erforderliche Atemluftmenge zur Verfügung zu stellen, ist mittig im Verneblungsraum ein zylindrischer Zuluftkamin 5 vorgesehen, durch den Umgebungsluft in den Verneblungsraum 1 geführt wird. Das untere, in den Verneblungsraum ragende Ende des Zuluftkamins 5 liegt in der Nähe der Düsenöffnungen 3a der Zerstäuberdüse 3.

Der in Fig. 1 gezeigte Vernebler besitzt einen Verneblungsraumteiler 6, der den Verneblungsraum 1 in zwei Teile 1a und 1b unterteilt. Der Verneblungsraumteiler 6 besteht aus einem Prallabschnitt 6a und Leitabschnitten 6b, von denen in der Ansicht der Fig.1 nur einer erkennbar ist. Der Prallabschnitt 6a ist am unteren Ende des Zuluftkamins 5 um diesen herum angeordnet und läßt auf der der Öffnung 4a abgewandten Seite des Zuluftkamins 5 eine Durchtrittsöffnung 6c für den Flüssigkeitsnebel frei. Die Leitabschnitte 6b erstrecken sich in Längsrichtung des Zuluftkamins und von der Außenwand des Zuluftkamins 5 bis zur Innenwand des Verneblungsraumes 1. Ebenso erstreckt sich der Prallabschnitt 6a vom Zuluftkamin 5 bis zur Innenwand des Verneblungsraumes 1.

Erfindungsgemäß besitzt der Vernebler aus Fig. 1 zumindest eine im wesentlichen ebene Prallfläche 7, die auf der den Leitabschnitten 6b abgewandten Seite des Prallabschnitts 6a des Verneblungsraumteilers 6 angeordnet ist und die sich im wesentlichen senkrecht zum Prallabschnitt 6a erstreckt. Die Prallfläche 7 reicht an den beiden zum Prallabschnitt 6a senkrecht verlaufenden Kanten bis an die Innenwand des Verneblungsraumes 1. Wie in Fig. 1 gezeigt, liegt die Prallfläche 7 auf der Seite der Zerstäuberdüse, die der Öffnung 6c des Prallabschnitts 6a zugewandt ist.

Durch die Prallfläche 7 wird erreicht, daß zu große Flüssigkeitströpfchen sich sofort auf der Oberfläche der Prallfläche 7 niederschlagen und in den Flüssigkeitssammelbereich 2 zurückgeführt werden, bevor sie noch durch die Durchtrittsöffnung 6c des Prallabschnitts 6a in den oberen Bereich des Verneblungsraumes 1 gelangen können. Auf diese Weise wird ein Niederschlagen der zu großen Flüssigkeitströpfchen im oberen Bereich des Verneblungsraumes 1 verhindert, von wo die Flüssigkeit bislang nicht mehr in den Flüssigkeitssammelbereich 2 zurückgelangen konnte. Die Ausbeute an entnehmbarem Flüssigkeitsnebel oder Aerosol ist somit erhöht, da die sich niederschlagende und nicht in den Sammelbereich 2 zurückgeführte Menge der Flüssigkeit reduziert ist.

Bei dem in Fig. 1 gezeigten Vernebler ist eine zweite Prallfläche 8 auf der der Prallfläche 7 gegenüberliegenden Seite der Zerstäuberdüse 3 angeordnet. Die Oberfläche der Prallfläche 8 liegt näher an den Düsenöffnungen 3a der Zerstäuberdüse 3 als die Oberfläche des Prallabschnitts 6a. Durch die Anordnung der zweiten Prallfläche 8 wird das Niederschlagen und Zurückführen großer Flüssigkeitströpfchen unmittelbar nach der Erzeugung durch die Zerstäuberdüse 3 weiter unterstützt.

Die erfindungsgemäßen Prallflächen sorgen dafür, daß nur so kleine Flüssigkeitströpfchen in den oberen Bereich des Verneblers 1 gelangen, daß diese sich bei der sich einstellenden Flüssigkeitsnebel/Luft-Strömung kaum noch an der Innenwand des Verneblungsraumes 1 niederschlagen. Die sich zu beiden Seiten der Düse ausbreitenden Aerosolstrahlen werden durch die Prallflächen 7 und 8 auf der gesamten zur Verfügung stehenden Ausbreitungslänge geführt und so ein ausreichender Raum zur Mischung des Primäraerosols mit der durch den Zuluftkamin 5 eintretenden Zuluft gebildet. Dadurch ist sichergestellt, daß die abgegebene Aerosolrate nicht durch Abscheidung feiner Tröpfchen unnötig reduziert wird.

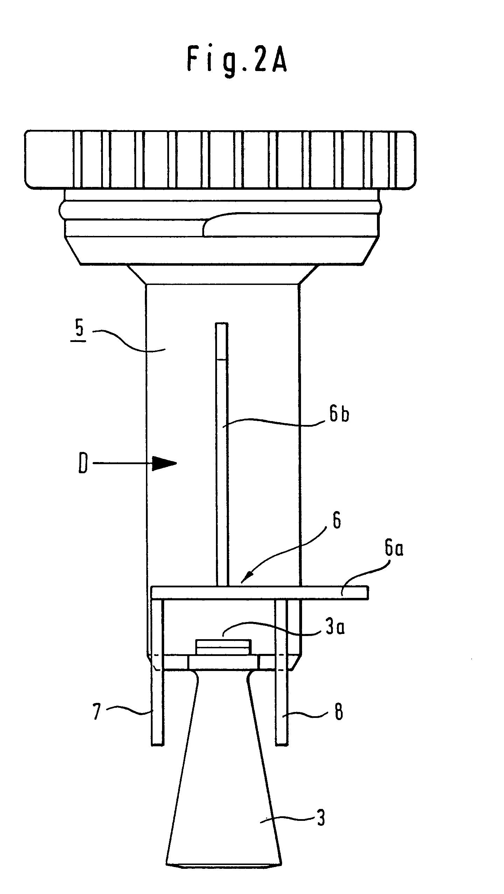

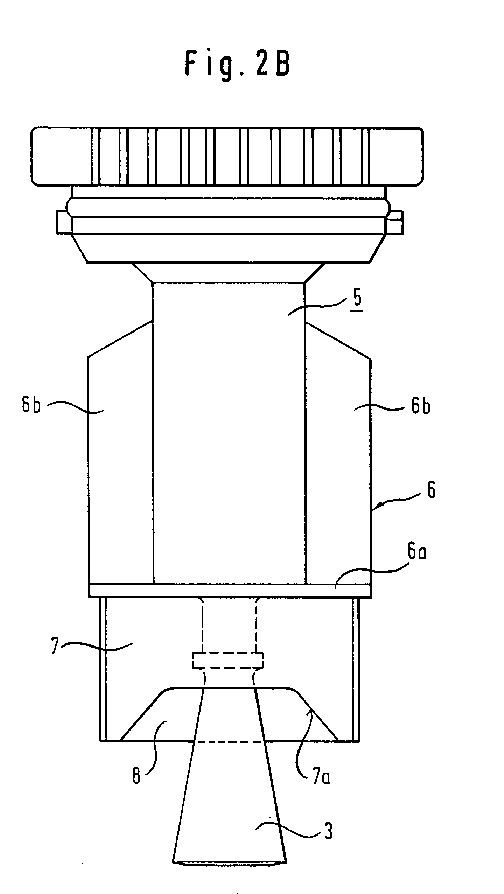

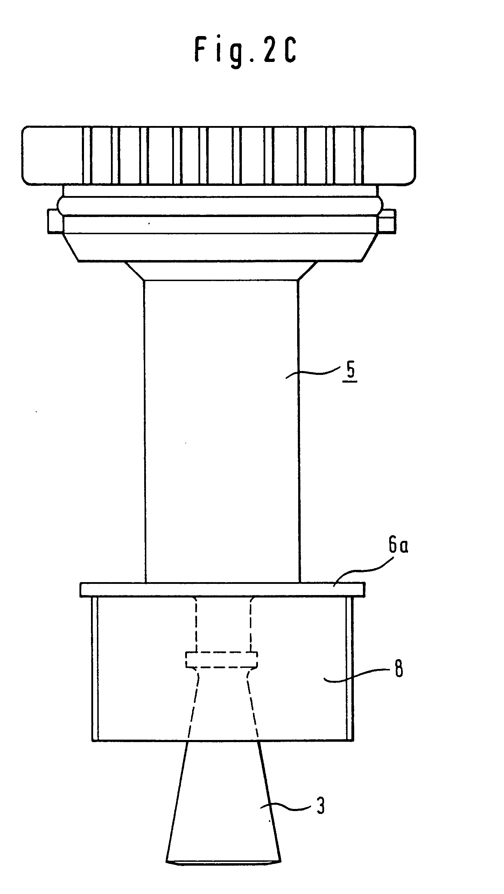

Die Fig. 2A bis 2C zeigen die erfindungsgemäßen Prallflächen in unterschiedlichen Ansichten. Dabei entspricht Fig. 2A der Ansicht der Prallflächen in Fig. 1. Auf beiden Seiten der Zerstäuberdüse ist eine Prallfläche 7 bzw. 8 angeordnet und erstreckt sich vom Prallabschnitt 6a senkrecht zur Zerstäuberdüse 3 hin. Auf der gegenüberliegenden Seite des Prallabschnitts 6a ist einer der beiden Leitabschnitte 6b des Verneblungsraumteilers 6 dargestellt. Der Leitabschnitt 6b verläuft in Richtung der Längsachse des Zuluftkamins 5. In Fig. 2B ist eine Ansicht in Richtung der Pfeile D in Fig. 2A gezeigt. In dieser Ansicht sind beide Leitabschnitte 6b am Zuluftkamin 5 erkennbar, die sich bis zum Prallabschnitt 6a erstrecken und gemeinsam mit diesem den Verneblungsraumteiler 6 bilden. Auf der den Leitabschnitten 6b gegenüberliegenden Seite des Prallabschnitts 6a ist die Prallfläche 7 sichtbar, die sich von der Oberfläche des Prallabschnitts 6a zur Zerstäuberdüse 3 hin erstreckt. Aus Fig. 2B ist ferner entnehmbar, daß in einer vorteilhaften Ausgestaltung die Prallfläche 7 an der dem Prallabschnitt 6a abgewandten Kante eine Ausnehmung 7a besitzt, die in der Ansicht der Fig. 2B einen Blick auf die dahinter liegende Prallfläche 8 des Ausführungsbeispiels freigibt. Durch die Ausnehmung 7a besteht die Möglichkeit der Anpassung der Wirkung der Prallfläche 7 an die Betriebsparameter des Verneblers. Demgegenüber ist die Prallfläche 8 bei dem hier beschriebenen Ausführungsbeispiel rechteckig, wie sich aus der Ansicht in Fig. 2C ergibt. In jedem Fall grenzen die Prallflächen 7 und 8 an den beiden schmalen Kanten an die Innenwand des Verneblerraumes 1.

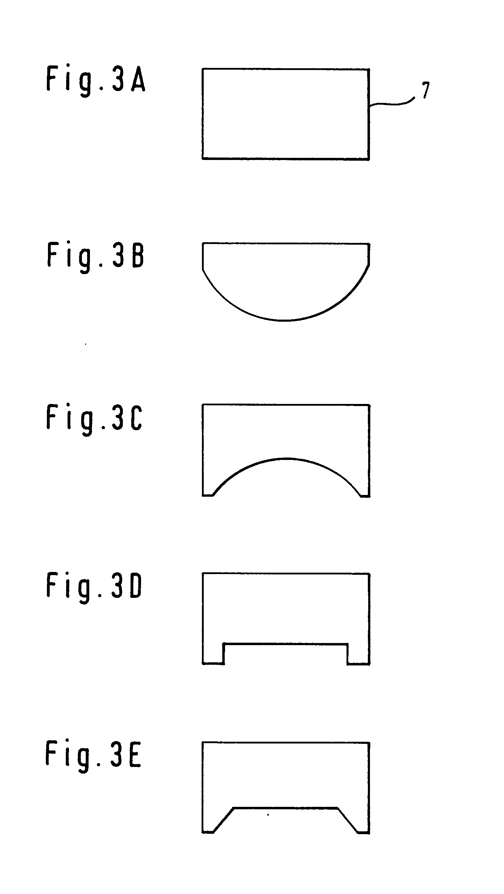

In den Fig. 3A bis 3F sind unterschiedliche Gestaltungen der vom Prallabschnitt 6a abgewandten Kante der Prallfläche 7 dargestellt. Im einfachsten Fall handelt es sich, wie in Fig. 3A gezeigt, um eine gerade Kante. Fig. 3B zeigt eine kreisförmig gebogene, hervorspringende Kante, Fig. 3C eine kreisbogenförmige Ausnehmung. Fig. 3D zeigt eine rechteckige Aussparung; durch Abschrägung der Schmalseiten der Aussparung gelangt man zur Ausgestaltung gem. Fig. 3E.

## Patentansprüche

1. Vernebler insbesondere zur Anwendung in der Inhalationstherapie mit
- einem Verneblungsraum (1), in dem mit Hilfe einer Zerstäuberdüse (3) ein Flüssigkeitsnebel erzeugt wird und der eine Öffnung (4a) für die Entnahme des Flüssigkeitsnebels aufweist,
- einem Zuluftkamin (5), der sich in den Verneblungsraum (1) hinein erstreckt, und
- einem Verneblungsraumteiler (6),
- - der in dem Verneblungsraum (1) derart angeordnet ist, daß der Verneblungsraum in einen ersten Teil (1a) und einen zweiten Teil (1b) unterteilt ist, so daß die sich im Verneblungsraum einstellende Flüssigkeitnebel/Luft-Strömung bei der Entnahme des Flüssigkeitsnebels von dem zweiten Teil (1b) in den ersten Teil (1a) verläuft, und
- - der einen Prallabschnitt (6a) umfaßt, der im kürzesten Weg zwischen der Öffnung (4a) des Verneblungsraumes (1) und dem Ort des Erzeugens des Flüssigkeitsnebels um den Zuluftkamin (5) herum angeordnet ist, so daß sich der Prallabschnitt (6a) im wesentlichen senkrecht zur Längsachse des Zuluftkamins (5) erstreckt, und der eine Durchtrittsöffnung (6c) für die Flüssigkeitsnebel/Luft-Strömung aufweist, und
- - der Leitabschnitte (6b) umfaßt, die sich beginnend am Prallabschnitt (6a) im wesentlichen in Richtung der Längsachse des Verneblungsraumes (1) zur Entnahmeöffnung (4a) und von dem Zuluftkamin (5) zur Innenwand des Verneblungsraumes (1) erstrecken,
**gekennzeichnet durch**
- zumindest eine Prallfläche (7),
-- die auf der den Leitabschnitten (6b) abgewandten Seite des Prallabschnitts (6a) des Verneblungsraumteilers (6) und auf der der Durchtrittsöffnung (6c) des Prallabschnitts (6a) zugewandten Seite der Zerstäuberdüse (3) angeordnet ist, und
-- die sich im wesentlichen senkrecht zum Prallabschnitt (6a) erstreckt.

2. Vernebler nach Anspruch 1, **dadurch gekennzeichnet, daß** eine weitere Prallfläche (8) auf der den Leitabschnitten (6b) abgewandten Seite des Prallabschnitts (6a) des Verneblungsraumteilers (6) und auf der der Durchtrittsöffnung (6c) des Prallabschnitts (6a) abgewandten Seite der Zerstäuberdüse (3) angeordnet ist und sich im wesentlichen senkrecht zum Prallabschnitt (6a) erstreckt.

3. Vernebler nach Anspruch 1 oder 2, **dadurch gekennzeichnet, daß** zumindest eine der Prallflächen (7, 8) eine rechteckige Grundform aufweist.

4. Vernebler nach Anspruch 3, **dadurch gekennzeichnet,**
**daß** die zum Prallabschnitt (6a) senkrecht verlaufenden Kanten der Prallflächen (7, 8) bis an die Innenwand des Verneblungsraumes (1) heranreichen.

5. Vernebler nach einem der Ansprüche 1 bis 4, **dadurch gekennzeichnet, daß** die vom Prallabschnitt (6a) abgewandte Kante zumindest des einen Prallabschnitts (7) eine kreisbogenförmige oder rechteckige Ausnehmung aufweist.

6. Vernebler nach einem der Ansprüche 1 bis 4, **dadurch gekennzeichnet, daß** die vom Prallabschnitt (6a) abgewandte Kante zumindest des einen Prallabschnitts (7) eine rechteckige Ausnehmung (7a) mit abgeschrägten Schmalseiten aufweist.

7. Vernebler nach einem der Ansprüche 1 bis 4, **dadurch gekennzeichnet, daß** die vom Prallabschnitt (6a) abgewandte Kante zumindest des einen Prallabschnitts (7) kreisbogenförmig hervorspringt.

## Claims

1. Nebuliser in particular for use in inhalation therapy with
- a nebulisation chamber (1) in which a liquid mist is produced by means of an atomiser nozzle (3) and which comprises an opening (4a) for extraction of the liquid mist,
- an air supply flue (5) which extends into the nebulisation chamber (1), and
- a nebulisation chamber divider (6)
- which is arranged in the nebulisation chamber (1) in such a way that the nebulisation chamber is divided into a first portion (1a) and a second portion (1b), so that the liquid mist/air stream arising in the nebulisation chamber passes from the second portion (1b) into the first portion (1a) on extraction of the liquid mist, and
- which includes a baffle section (6a) which is arranged around the air supply flue (5) on the shortest path between the opening (4a) of the nebulisation chamber (1) and the point of producing the liquid mist, so that the baffle section (6a) extends essentially perpendicularly to the longitudinal axis of the air supply flue (5), and which comprises a through-opening (6c) for the liquid mist/air stream, and
- which includes guide sections (6b) which, beginning at the baffle section (6a), extend essentially in the direction of the longitudinal axis of the nebulisation chamber (1) to the extraction opening (4a) and from the air supply flue (5) to the inner wall of the nebulisation chamber (1),
**characterised by**
- at least one baffle surface (7)
- which is arranged on the side of the baffle section (6a) of the nebulisation chamber divider (6) facing away from the guide sections (6b) and on the side of the atomiser nozzle (3) facing towards the through-opening (6c) of the baffle section (6a), and
- which extends essentially perpendicularly to the baffle section (6a).

2. Nebuliser according to claim 1, **characterised in that** an additional baffle surface (8) is arranged on the side of the baffle section (6a) of the nebulisation chamber divider (6) facing away from the guide sections (6b) and on the side of the atomiser nozzle (3) facing away from the through-opening (6c) of the baffle section (6a), and extends essentially perpendicularly to the baffle section (6a).

3. Nebuliser according to claim 1 or 2, **characterised in that** at least one of the baffle surfaces (7, 8) has a basic rectangular shape.

4. Nebuliser according to claim 3, **characterised in that** the edges of the baffle surfaces (7, 8) running perpendicularly to the baffle section (6a) extend as far as the inner wall of the nebulisation chamber (1).

5. Nebuliser according to any of claims 1 to 4, **characterised in that** the edge at least of one baffle section (7) facing away from the baffle section (6a) comprises an arcuate or rectangular recess.

6. Nebuliser according to any of claims 1 to 4, **characterised in that** the edge at least of one baffle section (7) facing away from the baffle section (6a) comprises a rectangular recess (7a) with bevelled narrow sides.

7. Nebuliser according to any of claims 1 to 4, **characterised in that** the edge at least of one baffle section (7) facing away from the baffle section (6a) protrudes arcuately.

## Revendications

1. Nébuliseur notamment destiné à l'utilisation en aérosolthérapie, comprenant
- une chambre de nébulisation (1) dans laquelle un aérosol de liquide est engendré à l'aide d'une buse d'atomisation (3), et qui est pourvue d'un orifice (4a) pour le prélèvement dudit aérosol de liquide,
- un conduit (5) d'amenée d'air, pénétrant à l'intérieur de la chambre de nébulisation (1), et
- un séparateur (6) de la chambre de nébulisation
- - qui est disposé, dans ladite chambre de nébulisation (1), de façon telle que ladite chambre de nébulisation soit scindée en une première partie (1a) et en une seconde partie (1b), si bien que le courant d'air/aérosol de liquide, s'instaurant dans la chambre de nébulisation, circule de la seconde partie (1b) vers la première partie (1a) lors du prélèvement de l'aérosol de liquide ;
- - qui renferme une chicane (6a) entourant périphériquement le conduit (5) d'amenée d'air sur le trajet le plus court entre l'orifice (4a) de la chambre de nébulisation (1) et le site de génération de l'aérosol de liquide, de sorte que ladite chicane (6a) s'étend pour l'essentiel perpendiculairement à l'axe longitudinal du conduit (5) d'amenée d'air, et comporte un orifice (6c) de passage du courant d'air/aérosol de liquide ; et
- - qui comprend des zones directrices (6b) s'étendant vers l'orifice de prélèvement (4a) à partir de la chicane (6a), pour l'essentiel dans la direction de l'axe longitudinal de la chambre de nébulisation (1), et vers la paroi intérieure de ladite chambre de nébulisation (1) à partir du conduit (5) d'amenée d'air,
**caractérisé par**
- au moins une surface déflectrice (7)
- - située sur le côté de la chicane (6a) du séparateur (6) de la chambre de nébulisation qui est tourné à l'opposé des zones directrices (6b), et sur le côté de la buse d'atomisation (3) qui est tourné vers l'orifice de passage (6c) de ladite chicane (6a) ; et
- - s'étendant pour l'essentiel perpendiculairement à ladite chicane (6a).

2. Nébuliseur selon la revendication 1, **caractérisé par le fait qu'**une surface déflectrice supplémentaire (8) est située sur le côté de la chicane (6a) du séparateur (6) de la chambre de nébulisation qui est tourné à l'opposé des zones directrices (6b), et sur le côté de la buse d'atomisation (3) qui est tourné à l'opposé de l'orifice de passage (6c) de ladite chicane (6a), et s'étend pour l'essentiel perpendiculairement à ladite chicane (6a).

3. Nébuliseur selon la revendication 1 ou 2, **caractérisé par le fait qu'**au moins l'une des surfaces déflectrices (7, 8) présente une configuration de base rectangulaire.

4. Nébuliseur selon la revendication 3, **caractérisé par le fait que** les bords des surfaces déflectrices (7, 8), qui sont orientés perpendiculairement à la chicane (6a), s'étendent jusqu'à la paroi intérieure de la chambre de nébulisation (1).

5. Nébuliseur selon l'une des revendications 1 à 4, **caractérisé par le fait que** le bord d'au moins l'une (7) des zones déflectrices, qui est tourné à l'opposé de la chicane (6a), présente un évidement en forme d'arc de cercle ou de rectangle.

6. Nébuliseur selon l'une des revendications 1 à 4, **caractérisé par le fait que** le bord d'au moins l'une (7) des zones déflectrices, qui est tourné à l'opposé de la chicane (6a), est muni d'un évidement rectangulaire (7a) dont les petits côtés sont biseautés.

7. Nébuliseur selon l'une des revendications 1 à 4, **caractérisé par le fait que** le bord d'au moins l'une (7) des zones déflectrices, qui est tourné à l'opposé de la chicane (6a), fait saillie sous la forme d'un arc de cercle.
